# FASCICULE DE BREVET EUROPEEN

(11) **EP 2 049 471 B1**
(45) Date de publication et mention de la délivrance du brevet: **13.07.2016**
(21) Numéro de dépôt: 07823334.3
(22) Date de dépôt: 25.07.2007
(51) Int. Cl.: C07C 303/00, C07C 309/06

(54) **PROCEDE DE PREPARATION D'UN ANHYDRIDE D'ACIDE SULFONIQUE**
VERFAHREN ZUR HERSTELLUNG EINES SULFONSÄURE-ANHYDRIDS
PROCESS FOR PREPARING A SULPHONIC ACID ANHYDRIDE

(30) Priorité: 27.07.2006 FR 0606881
(43) Date de publication de la demande: 22.04.2009
(73) Titulaire: Rhodia Opérations, 75009 Paris (FR)
(72) Inventeur: BESSON, Bernard, F-01700 Les Echets (FR)
(74) Mandataire: Dutruc-Rosset, Marie-Claude
(86) Numéro de dépôt international: PCT/FR2007/001276
(87) Numéro de publication internationale: WO 2008/012433

(56) Documents cités:
- WO-A-01/66516
- US-A- 5 808 149
- M. VIVEKANANDA BHATT ET AL.: "Aspects of tautomerism: Part X - Neighbouring group effects on the structure & reactivity patterns of acid chlorides." INDIAN JOURNAL OF CHEMISTRY, vol. 19b, 1980, pages 473-486, XP009083650 cité dans la demande

## Description

La présente invention a pour objet un procédé de préparation d'un anhydride d'acide sulfonique.

Elle concerne la préparation d'un anhydride symétrique ou mixte.

Elle vise plus particulièrement la préparation d'anhydride dérivé d'un superacide sulfonique.

Plus spécifiquement, l'invention vise la préparation d'anhydride trifluorométhanesulfonique dénommé également « anhydride triflique ».

Les superacides sulfoniques, c'est-à-dire ceux dont la constante de Hammett est au moins égale à 12, avantageusement à 13, sont de plus en plus utilisés comme intermédiaires de synthèse, et, notamment, pour former des groupes partants particulièrement réactifs.

Ces acides sulfoniques superacides sont en général des acides sulfoniques dont le soufre porte une fonction fluorée, en général un groupe portant un motif difluorométhylène reliant le soufre de la fonction sulfonique au reste de la molécule de l'acide.

Pour greffer ces groupes sulfonyle on peut utiliser les halogénures de sulfonyle. Toutefois, ces halogénures de sulfonyle présentent certaines difficultés d'emploi. Le groupe fluorure de sulfonyle est relativement peu réactif. Le chlorure et le bromure ont des propriétés oxydantes, c'est-à-dire respectivement chlorantes ou bromantes, qui les rendent difficiles d'emploi pour un grand nombre d'applications.

C'est pourquoi l'anhydride, qu'il soit symétrique, ou mixte, reste un moyen de greffer des groupes sulfonyle, sans ces inconvénients.

Toutefois, la synthèse de ces anhydrides sulfoniques reste très difficile, et la seule technique réellement efficace trouvée à ce jour réside dans la mise en contact des acides sulfoniques visés par la présente invention avec de l'anhydride phosphorique (P₂O₅). On peut se référer notamment aux brevets US 5 004 829 et US 5 808 149.

La réaction de déshydratation entre deux molécules d'acide sulfonique marche bien mais la quantité d'acide phosphorique et la difficulté de choisir un solvant adéquat rendent l'opération particulièrement difficile et coûteuse dans sa mise en oeuvre, notamment en raison des problèmes d'agitation qu'il convient de régler dans un milieu qui est particulièrement agressif.

L'objectif de la présente invention est de fournir un procédé palliant les inconvénients précités.

Il a maintenant été trouvé et c'est ce qui constitue l'objet de la présente invention, un procédé de préparation d'un anhydride d'acide sulfonique caractérisé par le fait qu'il comprend la réaction d'un acide sulfonique ou d'un mélange de deux acides sulfoniques avec un réactif présentant la tautomérie pseudohalogénure d'acide avec au moins un atome de carbone engagé dans la tautomérie qui porte deux atomes d'halogène.

Dans le présent texte, on entend par « halogène », le fluor, le chlore ou le brome et plus particulièrement l'atome de chlore.

On entend par « groupe, halophore », un atome de carbone qui porte un ou deux ou trois atomes d'halogène.

Par « groupe gem-dihalogéné », on entend un groupe avec deux atomes d'halogène sur le même atome de carbone.

Conformément au procédé de l'invention, il est possible de former une fonction anhydride sulfonique à partir de groupes sulfoniques par élimination d'une molécule d'eau grâce à la présence d'une molécule présentant la tautomérie pseudohalogénure d'acide.

De telles molécules sont bien décrites dans la littérature, en particulier par M. Vivekananda Bhatt et al (Indian Journal of Chemistry, 19B, June 1980, pp. 437-486).

Ainsi, intervient dans la procédé de l'invention, un réactif dont la caractéristique est d'avoir dans les conditions réactionnelles une forme tautomère de type pseudohalogénure c'est-à-dire une structure cyclique.

Un composé organique peut présenter pour une même formule chimique, une ou plusieurs structures électroniques qui sont en équilibre.

Les tautomères sont des composés organiques interchangeables par tautomérisation.

La tautomérie pseudohalogénure d'acide à partir d'un halogénure d'acide est la création d'un composé cyclique comprenant un pont oxygène entre un groupe gem-dihalogéné et un groupe carbonyle (ou sulfonyle) ou un groupe gem-dihalogéné et un groupe halophore qui est un atome de carbone monohalogéné ou un groupe gem-dihalogéné.

Le réactif de l'invention, dénommé de manière simplifiée dans la suite du texte par « réactif », doit posséder une forme tautomère de type pseudohalogénure mais également l'atome de carbone qui est engagé dans la tautomérie porte deux atomes d'halogène.

Conformément au procédé de l'invention, le réactif doit posséder une forme tautomère de type cyclique qui comprend le motif suivant en équilibre avec sa forme tautomère : 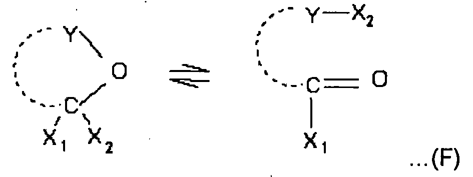 dans ladite formule (F),
- Y représente l'un des groupes suivants :
   . CO,
   .CX₁'X₂',
- X₁, X₂, représentent un atome d'halogène, de préférence X₁, X₂ représentent tous deux un atome de chlore,
- X₁', X₂', représentent un atome d'hydrogène ou un atome d'halogène, et au plus l'un de X₁' et X₂' représentent un atome d'hydrogène,
- le demi cercle symbolisé par des tirets représente un groupe divalent comprenant de 2 à 4 atomes de carbone :
   qui est une chaîne hydrocarbonée linéaire comprenant au moins une double liaison
   ou qui fait partie d'un ou de deux cycles insaturés ou aromatiques.

Par « cycle insaturé », on entend dans le présent texte, un cycle hydrocarboné ayant 5 ou 6 atomes de carbone, de préférence 6 atomes, comprenant une ou deux doubles liaisons.

Par « cycle aromatique », on entend dans le présent texte, un cycle ou un bicycle hydrocarboné aromatique ayant de 6 à 12 atomes de carbone, de préférence un cycle benzénique.

Dans la formule (F), le demi cercle symbolise les atomes qui sont présents dans l'hétérocycle permettant d'obtenir la tautomérie.

Ainsi, l'invention n'exclut pas d'autres structures aliphatiques ou aromatiques dès lors que l'on puisse obtenir la structure hétérocyclique permettant d'obtenir la tautomérie.

De même l'invention n'exclut pas la présence de substituants sur les liaisons vacantes des doubles liaisons ou sur le cycle dans la mesure où ils ne réagissent pas. On peut citer notamment les groupes alkyle ou alkoxy C₁-C₄ ou les atomes d'halogène, de préférence de chlore ou de brome.

Il apparaît que les fonctions présentes dans le motif de formule (F) doivent être topologiquement proches de manière à être jointes par ledit pont oxygène formant ainsi un hétérocycle d'au plus 7 atomes, préférentiellement de 5 ou 6 atomes.

Avantageusement, dans une chaîne hydrocarbonée aliphatique, le groupe halophore CX₁X₂ est en position γ ou δ par rapport à Y, de préférence en position γ.

Dans la formule (F), le demi-cercle représenté par des tirets représente préférentiellement une chaîne aliphatique comprenant une ou deux doubles liaisons, de préférence une liaison éthylénique.

Lorsque une ou deux doubles liaisons sont incluses dans un ou deux cycles, dans la formule (F), le demi-cercle représenté par des tirets représente préférentiellement un groupe aromatique tel qu'un groupe 1,2-phénylène ou un groupe 1,2-naphtyldiyle ou un groupe 1,8-naphtyldiyle.

Comme mentionné précédemment, les liaisons vacantes de la double liaison ou les positions libres des réactifs comprenant un cycle peuvent porter un substituant par exemple des atomes d'halogène.

Ainsi, une première classe de réactifs préférés répondent aux formules suivantes en équilibre avec leurs formes tautomères : 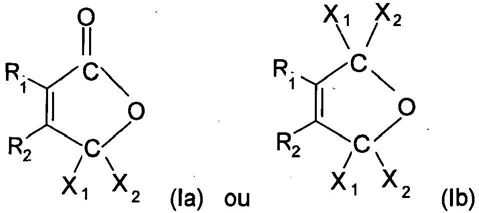 dans lesdites formules :
- R₁, R₂, représentent un atome d'hydrogène, un groupe alkyle linéaire ou ramifié ayant de 1 à 4 atomes de carbone, un atome d'halogène,
- ou R₁ et R₂, sont liés pour former un groupe 1,2-phénylène ou un groupe 1,2-naphtyldiyle,
- X₁, X₂, représentent un atome d'halogène, de préférence X₁, X₂ représentent tous deux un atome de chlore.

Ainsi, les réactifs préférés mis en oeuvre dans le procédé de l'invention répondent à la formule (la) ou (Ib) (et leurs formes tautomères) dans laquelle R₁ et R₂ représentent un atome d'hydrogène ou un groupe méthyle ou R₁ et R₂ sont liés pour former l'un des groupes suivants :

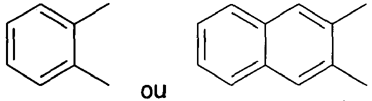

Comme mentionné précédemment, l'invention inclut le cas où les cycles aromatiques portent des substituants. N'importe quel substituant peut être présent dans la mesure où ils ne gênent pas l'obtention du produit souhaité. Des exemples ont été donnés ci-dessus.

On donne ci-après des exemples de réactifs présents lors de la réaction :

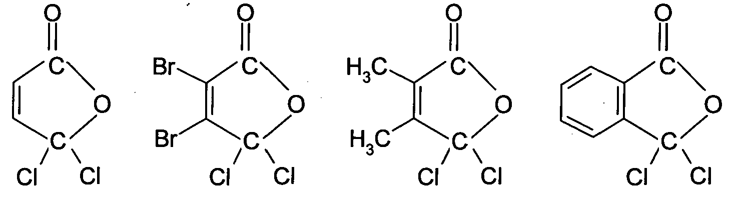

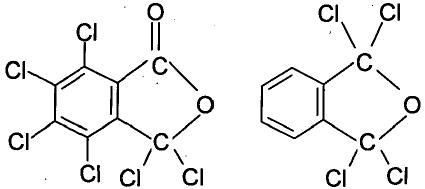

Le réactif qui est préféré, comprend le motif représenté par la formule suivante en équilibre avec sa forme tautomère : 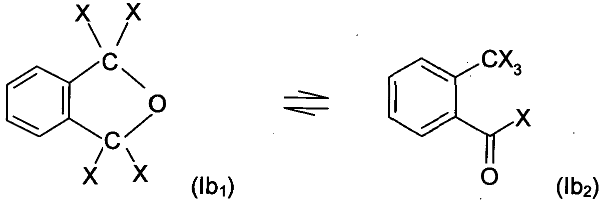 dans lesdites formules, X est un atome d'halogène, de préférence un atome de chlore.

Une autre classe de réactifs préférés sont ceux dans lesquels les groupes fonctionnels Y et CX₁X₂ sont portés par un atome de carbone appartenant à deux cycles benzéniques différents mais qui sont ortho-condensés.

Un exemple de ce type de réactif est donné par celui qui répond à la formule (Ic) suivante :

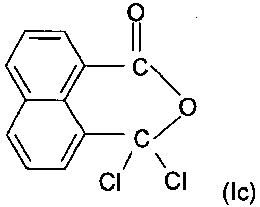

Les différents réactifs sont des produits connus, disponibles dans le commerce ou décrits dans la littérature notamment par M. Vivekananda Bhatt et al (loc. cit).

Pour ce qui est de l'acide sulfonique, il peut être représenté par la formule suivante :

R_{f}-SO₃H (II)

dans ladite formule :
- R_{f} représente un groupe hydrocarboné ayant de 1 à 20 atomes de carbone comprenant ou non au moins un atome d'halogène, de préférence un atome de fluor,

Dans la formule (II), R_{f} représente préférentiellement un groupe hydrocarboné ayant de 1 à 20 atomes de carbone, et plus particulièrement :
- un groupe alkyle, linéaire ou ramifié, ayant de 1 à 12 atomes de carbone,
- un groupe alcényle ou alcynyle, linéaire ou ramifié, ayant de 2 à 12 atomes de carbone,
- un groupe cycloalkyle ou cycloalcényle, ayant de 3 à 8 atomes de carbone,
- un groupe aryle ayant de 6 à 20 atomes de carbone, de préférence un groupe phényle ou un groupe naphtyle,
- un groupe arylalkyle ayant de 7 à 20 atome de carbone, de préférence un groupe benzyle.

Les différents groupes précités, alkyle, alcényle, alcynyle, cycloalkyle, aryle ou arylalkyle peuvent être substitués par un substituant quelconque dans la mesure où ils ne gênent pas la réaction. Comme exemples plus particuliers de substituants, on peut mentionner entre autres, les atomes d'halogène, de préférence fluor, chlore, brome.

Plus précisément, dans la formule (II), R_{f} représente un groupe hydrocarboné ayant de 1 à 20 atomes de carbone et plus particulièrement :
- un groupe alkyle en C₁ à C₁₀, de préférence en C₁ à C₄, et plus préférentiellement un groupe méthyle ou éthyle,
- un groupe alkyle en C₁ à C₁₀, de préférence en C₁ à C₄, mono-, poly- ou per- halogéné ayant de 1 à 23 atomes d'halogène, de préférence un groupe CF₃,
- un groupe cycloalkyle ayant de 3 à 8 atomes de carbone, de préférence un groupe cyclohexyle,
- un groupe cycloalkyle éventuellement mono-, poly- ou per-halogéné, ayant de 3 à 8 atomes de carbone,
- un groupe phényle,
- un groupe phényle mono-, poly- ou per-halogéné,
- un groupe phényle substitué par au moins un groupe alkyle en C₁ à C₁₀, de préférence en C₁ à C₄, éventuellement mono-, poly- ou per-halogéné ou un groupe nitro ou nitrile ;
- un groupe aryle éventuellement mono-, poly- ou per-halogéné, ayant de 6 à 12 atomes de carbone.

Plus préférentiellement, R_{f} représente représente un groupe méthyle, phényle, tolyle, un groupe CF₃, C₄F₉ ou un groupe phényle substitué par un ou plusieurs atomes d'halogène de préférence de fluor, ou par un ou plusieurs groupes alkyle en C₁-C₂ mono-, poly- ou per- fluoré.

Comme exemples d'acides sulfoniques, on peut mentionner notamment les acides halogénosulfoniques, de préférence l'acide fluorosulfonique, l'acide chlorosulfonique, l'acide trifluorométhanesulfonique, l'acide perfluoroéthanesulfonique, l'acide perfluorobutanesulfonique, l'acide perfluorooctanesulfonique ; les acides sulfoniques aliphatiques de préférence l'acide méthanesulfonique, l'acide éthanesulfonique ; les acides sulfoniques aromatiques de préférence l'acide benzènesulfonique, les acides toluènesulfoniques, les acides naphtalènesulfoniques.

Parmi ces acides, on met en oeuvre préférentiellement l'acide trifluorométhanesulfonique, l'acide paratoluènesulfonique, l'acide méthanesulfonique, l'acide benzènesulfonique.

Conformément au procédé de l'invention, on fait réagir le réactif défini selon l'invention avec l'acide sulfonique.

La quantité de réactif mis en oeuvre est généralement fonction du nombre de groupes gem-dihalogénés présents dans la formule (I) et engagés dans la réaction de tautomérie.

Ainsi, dans le cas d'un réactif comprenant un groupe gem-dihalogéné comme dans la formule (la) ou (Ic), la quantité de ce dernier mis en oeuvre est telle que le rapport entre le nombre de moles de réactif comprenant un groupe gem-dihalogéné, de préférence de formule (la) ou (Ic) et le nombre de moles d'acide sulfonique est choisi avantageusement entre 0,5 et 1, de préférence entre 0,5 et 0,7.

Lorsque le réactif comprend deux groupes gem-dihalogénés, la quantité de ce dernier mis en oeuvre est telle que le rapport entre le nombre de moles de réactif comprenant deux groupes gem-dihalogénés, de préférence de formule (Ib) et le nombre de moles d'acide sulfonique est choisi avantageusement entre 0,25 et 0,5, de préférence entre 0,25 et 0,35.

La réaction peut être menée en présence de solvants inertes vis-à-vis des deux réactifs et des produits de la réaction.

Comme exemples de solvants susceptibles d'être utilisés, on peut citer notamment les hydrocarbures aliphatiques, cycloaliphatiques, aromatiques, de préférence l'hexane, le cyclohexane, le méthylcyclohexane, l'heptane, le dodécane, le benzène, le toluène, les xylènes, l'éthylbenzène, les diéthylbenzènes, les triméthylbenzènes, le cumène, le pseudocumène, les coupes pétrolières constituées de mélange d'alkylbenzènes notamment les coupes de type Solvesso^{®}.

On préfère toutefois conduire le procédé de l'invention en l'absence de solvants organiques.

La réaction est menée sous pression ordinaire mais il ne s'agit pas d'un point critique. La réaction est avantageusement conduite à une température comprise entre 80°C et 180°C, de préférence entre 100°C et 150°C.

La température optimale se situe entre 110°C et 140°C.

Le procédé est simple à mettre en oeuvre. On mélange le réactif et l'acide sulfonique dans un ordre quelconque généralement à température ambiante (le plus souvent entre 15°C et 25°C) puis l'on augmente la température jusqu'à dégagement de l'acide halohydrique, de préférence l'acide chlorhydrique qui se forme au cours de la réaction.

Un mode préféré consiste à porter le réactif à la température réactionnelle choisie, puis à y ajouter progressivement, de préférence par coulée, l'acide sulfonique.

Le procédé de l'invention peut être conduit en continu par exemple dans un appareil de distillation à court temps de séjour (comme par exemple l'appareil LUWA^{®}).

Il peut être conduit également d'une manière discontinue, dans un réacteur classique équipé de moyens d'agitation et de chauffage surmonté d'une colonne de distillation.

La température de la réaction peut être contrôlée, soit à l'aide d'un échangeur thermique, soit par circulation d'un fluide caloporteur dans une double enveloppe dont il serait muni.

Comme fluides caloporteurs convenant à l'invention, on peut mentionner notamment l'eau ou bien un solvant organique tel que choisi parmi les esters lourds d'acides carboxyliques (par exemple, le phtalate d'octyle), les éthers aromatiques comme l'oxyde de biphényle et/ou l'oxyde de benzyle, le diphényle, les terphényles, les autres polyphényles éventuellement partiellement hydrogénés, les huiles paraffiniques et/ou naphténiques, résidus de distillation du pétrole etc...

Le flux gazeux recueilli, en tête de colonne, comprenant de l'acide halohydrique est condensé par passage dans un ou plusieurs échangeurs thermiques refroidis par de l'eau, de l'eau glycolée ou bien tout autre caloporteur compatible avec la température de refroidissement choisie.

Conformément au procédé de l'invention, on obtient un anhydride sulfonique symétrique ou mixte qui peut être symbolisé par la formule (III) : 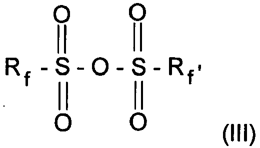 dans ladite formule,
- R_{f}' a la signification donnée pour R_{f},
- R_{f} est identique à R_{f}' dans le cas des anhydrides symétriques,
- et R_{f}' est différent de R_{f}' dans le cas des anhyrides mixtes.

La récupération de l'anhydride sulfonique formé à partir du milieu réactionnel relève de la compétence de l'Homme du Métier.

En fonction du point d'ébullition de l'anhydride sulfonique formé, plusieurs modes de récupération peuvent être envisagés.

Dans le cas de l'anhydride triflique, ce dernier constitue un flux gazeux avec l'acide halohydrique qui est récupéré par condensation dans un échangeur. L'acide halohydrique est envoyé sur une colonne d'abattage à la soude.

Si la température d'ébullition de l'anhydride sulfonique formé est supérieure à celle de la température de réaction, l'anhydride sulfonique reste dans le milieu réactionnel, en pied de distillation tandis que l'acide halohydrique est récupéré en tête de distillation.

Si l'anhydride sulfonique formé reste en pied de distillation, celui peut être récupéré subséquemment par tout moyen approprié en particulier par distillation, le plus souvent par distillation sous pression réduite.

On donne ci-après des exemples de réalisation donnés à titre illustratif.

Dans les exemples, les abbréviations utilisées ont les significations suivantes :
- RO = o-trichlorométhylchlorure de benzoyle,
- TA = acide trifluorométhanesulfonique = acide triflique,
- TAA = anhydride trifluorométhanesulfonique = anhydride triflique,
- PA = anhydride phtalique.

Le rendement (RR) correspond au rapport entre le nombre de moles de produit formées et le nombre de moles de substrat engagées.

### EXEMPLES

### Exemple 1

### Préparation de l'anhydride triflique.

On opère dans un réacteur de 250 ml chauffé par une double enveloppe et équipé d'une agitation mécanique centrale, d'une ampoule de coulée et d'un col de cygne alimentant une recette refroidie à 15°C et respirant à l'atmosphère via a) un piège à carboglace b) un flacon laveur contenant une solution de soude.

On charge 73 g de RO qui constitue le "pied" du mélange réactionnel : l'o-trichlorométhylchlorure de benzoyle mis en oeuvre est en équilibre avec sa forme tautomère pseudochlorure dans un rapport molaire 20/80.

On chauffe ce pied à 120°C.

On alimente progressivement 157 g d'acide triflique à partir de l'ampoule de coulée en 3 heures en maintenant la température de la masse réactionnelle entre 115 et 135°C.

Dès le début de la coulée on observe le dégagement d'HCl et après environ 15 min, on condense du TAA brut.

On finit l'épuisement du flux d'HCl en vapeurs organiques en les condensant dans le piège à carboglace.

Après la fin de l'alimentation on épuise le culot résiduel par 5 min de chauffage à 140°C. On arrête la réaction quand plus rien ne distille.

On recueille le TAA brut (condensat et piégeat), le culot réactionnel et la solution de lavage.

Ils sont analysés par les méthodes appropriées (RMN du Fluor, chromatographie ionique et potentiométrie).

Les résultats obtenus sont les suivants :
- RR en anhydride triflique : 91, 4 % en moles,
- RR en chlorure de trifluorométhylsulfonyle : 0,34 % en mole,
- RR en triflate de trifluorométhyle : 0,07 % en mole.

### Exemples 2 et 3

### Essais comparatifs A à O

On effectue une série d'essais de type qualitatif afin de mettre en évidence l'influence de la nature du réactif mis en oeuvre.

Avant de détailler les exemples et essais, on fait une description de l'appareillage utilisé et du protocole opératoire suivi.

### Appareillage utilisé.

L'appareillage utilisé est un four à gradient de température BUCHI^{®} (Glass oven B 580).

Il se présente sous la forme d'un four tubulaire en verre dans lequel est introduit un ballon réacteur monocol auquel sont connectés deux ballons récepteurs.

L'ensemble est solidaire et peut être mis en rotation par l'intermédiaire d'un moteur.

Les produits légers issus de la réaction sont piégés en sortie par de la carboglace dans deux ballons récepteurs.

L'émission éventuelle de gaz est contrôlée visuellement par compte-bulles.

### Mode opératoire :

Les réactifs sont pesés sous hotte dans le ballon réacteur (poids maxi de 10 g).

Le ratio molaire entre l'acide sulfonique et le réactif est précisé dans les tableaux suivants.

Les deux ballons récepteurs sont ajoutés en ligne et l'ensemble est maintenu par des clips métalliques.

Le ballon réacteur et le premier ballon récepteur sont insérés dans le four à gradient de température.

Le deuxième ballon récepteur est placé à l'extérieur est refroidi par de la carboglace.

L'ensemble des ballons est mis en rotation et la température du four est graduellement portée par pallier de 10 degrés jusqu'à une température légèrement inférieure à la température de distillation du composé le plus volatil.

La température est maintenue pendant trente minutes et l'on contrôle visuellement la formation de gaz et la distillation de composés piégeables.

En fin de maintien, la température est redescendue à l'ambiante et le distillat recueilli dans le ballon récepteur extérieur au four est analysé par RMN ou par chromatographie ionique.

### Essais A à K

Dans ces essais, on met en oeuvre comme acide sulfonique, l'acide triflique et comme réactifs, des chlorures d'acides mono- et difonctionnels.

Les résultats sont consignés dans le tableau (I).

**Tableau (I)**

| Réf. essai | Nature du réactif | Température d'ébullition | Ratio acide sulfonique/réactif | Distillation |
|---|---|---|---|---|
| A | Chlorure de benzoyle | 198°C | 2 | non |
| B | Chlorure de pivaloyle | 105°C | 1 | non |
| C | Chlorure de propionyle | 77 - 79°C | 1 | non |
| D | Chlorure d'acétyle | 52°C | 1 | non |
| E | Chlorure de trichloroacétyle | 115°C | 1 | non |
| F | Chlorure d'adipoyle | 106°C 2 mm Hg | 1 | non |
| G | Chlorure de succinyle | 190°C | 1 | non |
| H | Chlorure de malonyle | 54°C 19 mm Hg | 1 | non |
| I | Chlorure d'oxalyle | 62 - 63°C | 1 | non |
| J | Chlorure de téréphtaloyle | 266°C | 2 | non |
| K | Chlorure d'isophtaloyle | 276°C | 2 | non |

On n'observe pas de distillat. Il n'y a donc pas formation d'anhydride triflique.

### Essais L à N

Dans ces essais, on met en oeuvre comme acide sulfonique, l'acide triflique et comme réactif, un composé présentant une fonction trichlorométhyle.

Les résultats sont consignés dans le tableau (II).

**Tableau (II)**

| Réf. essai | Nature du réactif | Température d'ébullition | Ratio acide sulfonique/réactif | Distillation |
|---|---|---|---|---|
| L | 1,4-bis-trichlorométhyl-benzène | 312°C | 2 | non |
| M | Trichlorotoluène | 219-223°C | 2 | non |
| N | 4-chlorotrichloro-méthylbenzène | 245°C | 1 | non |

On n'observe pas de distillat. Il n'y a donc pas formation d'anhydride triflique.

### Essai O

Dans cet essai, on met en oeuvre comme acide sulfonique, l'acide triflique et comme réactif, un composé présentant un carbone gem dichloré non impliqué dans une tautomérie pseudo chlorure d'acide.

Les résultats sont consignés dans le tableau (III).

**Tableau (III)**

| Réf. essai | Nature du réactif | Ratio acide sulfonique/réactif | Distillation |
|---|---|---|---|
| O | 2-2 dichlorobenzodioxole | 1 | non |

On n'observe pas de distillat. Il n'y a donc pas formation d'anhydride triflique.

### Exemple 2

Dans cet exemple, on met en oeuvre comme acide sulfonique, l'acide triflique et comme réactif, un composé présentant au moins un atome de carbone gem dichloré impliqué dans une tautomérie pseudochlorure d'acide.

Les résultats sont consignés dans le tableau (IV) :

**Tableau (IV)**

| Réf. essai | Nature du réactif | Température d'ébullition | Ratio acide sulfonique/réactif | Distillation |
|---|---|---|---|---|
| 2 | Chlorure de 2-trichlorométhylbenzoyle | >150°C | 2 | oui |

Le distillat recueilli est analysé par spectroscopie de résonance magnétique du fluor. Il est constitué essentiellement d'anhydride triflique.

### Exemple 3

Dans cet exemple, l'acide sulfonique mis en oeuvre est l'acide méthanesulfonique et le réactif est celui de l'exemple 2.

Les résultats sont consignés dans le tableau (V) :

**Tableau (V)**

| Réf. essai | Nature du réactif | Température d'ébullition | Ratio acide sulfonique/réactif |
|---|---|---|---|
| 3 | Chlorure de 2-trichlorométhylbenzoyle | 167°C 10 mm de Hg | 2 |

Dans cet exemple, il n'y a pas de distillat mais on a observé un dégagement gazeux acide et l'analyse du produit résiduel blanc dans le ballon réacteur montre qu'il est constitué pour partie par de l'anhydride méthanesulfonique et par de l'anhydride phtalique.

## Revendications

1. Procédé de préparation d'un anhydride d'acide sulfonique, **caractérisé par le fait qu'**il comprend la réaction, conduite à une température comprise entre 80°C et 180°C, d'un acide sulfonique ou d'un mélange de deux acides sulfoniques avec un réactif présentant dans les conditions réactionnelles la tautomérie pseudohalogénure d'acide avec au moins un atome de carbone engagé dans la tautomérie qui porte deux atomes d'halogène ; ledit réactif possédant le motif suivant en équilibre avec sa forme tautomère : 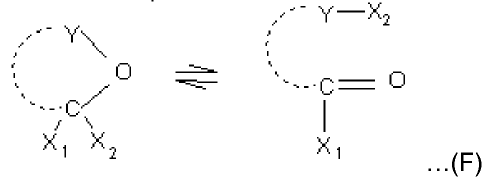 dans ladite formule (F),
- Y représente l'un des groupes suivants :
. CO,
. CX₁'X₂',
- X₁, X₂, représentent un atome d'halogène,
- X₁', X₂', représentent un atome d'hydrogène ou un atome d'halogène, et au plus l'un de X₁' et X₂' représentent un atome d'hydrogène,
- le demi cercle symbolisé par des tirets représente un groupe divalent comprenant de 2 à 4 atomes de carbone :
. qui est une chaîne hydrocarbonée linéaire comprenant une ou deux doubles liaisons,
. ou qui fait partie d'un ou de deux cycles insaturés ou aromatiques.

2. Procédé selon la revendication 1, **caractérisé par le fait que** l'halogène est l'atome de chlore.

3. Procédé selon la revendication 1, **caractérisé par le fait que** le demi-cercle représenté par des tirets dans la formule (F) représente une liaison éthylénique ou un groupe 1,2-phénylène ou un groupe 1,2-naphtyldiyle ou un groupe 1,8-naphtyldiyle.

4. Procédé selon la revendication 1, **caractérisé par le fait que** le demi-cercle représenté par des tirets dans la formule (F), est une chaîne hydrocarbonée aliphatique dans laquelle le groupe halophore CX₁X₂ est en position γ ou δ par rapport à Y.

5. Procédé selon l'une des revendications 1 à 4, **caractérisé par le fait que** les réactifs répondent aux formules suivantes en équilibre avec leurs formes tautomères : 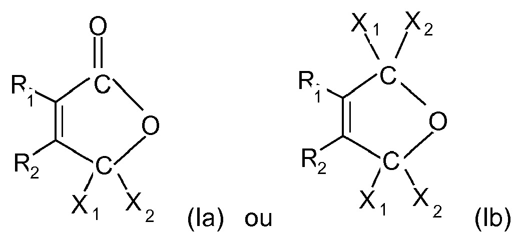 dans lesdites formules :
- R₁, R₂, représentent un atome d'hydrogène, un groupe alkyle linéaire ou ramifié ayant de 1 à 4 atomes de carbone, un atome d'halogène,
- ou R₁ et R₂, sont liés pour former un groupe 1,2-phénylène ou un groupe naphtyldiyle-1,2,
- X₁, X₂, représentent un atome d'halogène.

6. Procédé selon la revendication 5, **caractérisé par le fait que** le réactif répond à la formule (la) ou (Ib) (et leurs formes tautomères) dans laquelle R₁, R₂ représentent un atome d'hydrogène ou un groupe méthyle ou R₁ et R₂ sont liés pour former l'un des groupes suivants :

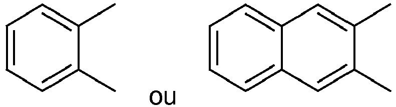

7. Procédé selon l'une des revendications 5 et 6, **caractérisé par le fait que** le réactif répond à l'une des formules suivantes :

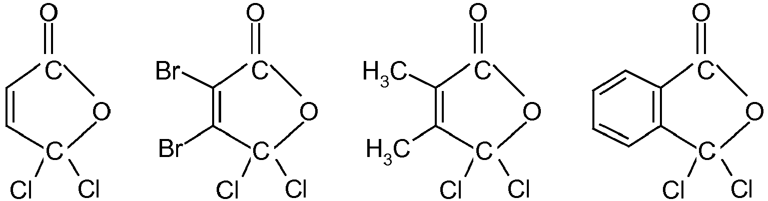

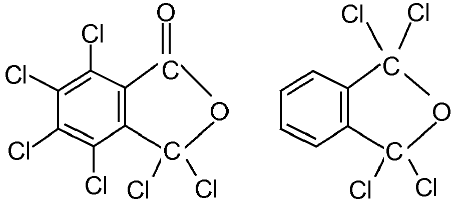

8. Procédé selon la revendication 1, **caractérisé par le fait que** le réactif comprend le motif représenté par la formule suivante en équilibre avec sa forme tautomère : 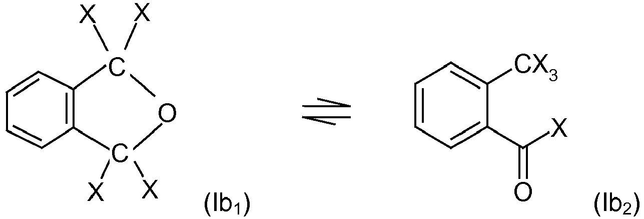 dans lesdites formules, X est un atome d'halogène.

9. Procédé selon la revendication 1, **caractérisé par le fait que** le réactif comprend des groupes fonctionnels Y et CX₁X₂ qui sont portés par un atome de carbone appartenant à deux cycles benzéniques différents mais qui sont ortho-condensés.

10. Procédé selon la revendication 9, **caractérisé par le fait que** le réactif répond à la formule suivante :

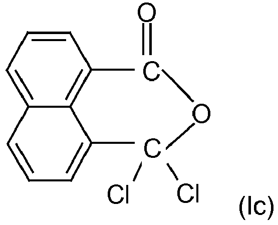

11. Procédé selon l'une des revendications 1 à 10, **caractérisé par le fait que** l'acide sulfonique répond à la formule suivante :
R_{f}-SO₃H (II)
dans ladite formule :
- R_{f} représente un groupe hydrocarboné ayant de 1 à 20 atomes de carbone comprenant ou non au moins un atome d'halogène.

12. Procédé selon la revendication 11, **caractérisé par le fait que** le groupe hydrocarboné R_{f} est choisi parmi :
- un groupe alkyle, linéaire ou ramifié, ayant de 1 à 12 atomes de carbone,
- un groupe alcényle ou alcynyle, linéaire ou ramifié, ayant de 2 à 12 atomes de carbone,
- un groupe cycloalkyle ou cycloalcényle, ayant de 3 à 8 atomes de carbone,
- un groupe aryle ayant de 6 à 20 atomes de carbone,
- un groupe arylalkyle ayant de 7 à 20 atome de carbone.

13. Procédé selon la revendication 11, **caractérisé par le fait que** l'acide sulfonique répond à la formule (II) dans laquelle R_{f} représente :
- un groupe alkyle en C₁ à C₁₀,
- un groupe alkyle en C₁ à C₁₀ mono-, poly- ou per- halogéné ayant de 1 à 23 atomes d'halogène,
- un groupe cycloalkyle ayant de 3 à 8 atomes de carbone,
- un groupe cycloalkyle éventuellement mono-, poly- ou per-halogéné, ayant de 3 à 8 atomes de carbone,
- un groupe phényle,
- un groupe phényle mono-, poly- ou per-halogéné,
- un groupe phényle substitué par au moins un groupe alkyle en C₁ à C₁₀ éventuellement mono-, poly- ou per- halogéné ou un groupe nitro ou nitrile ;
- un groupe aryle éventuellement mono-, poly- ou per-halogéné, ayant de 6 à 12 atomes de carbone.

14. Procédé selon la revendication 13, **caractérisé par le fait que** l'acide sulfonique répond à la formule (II) dans laquelle R_{f} représente un groupe méthyle, phényle, tolyle, un groupe CF₃, C₄F₉ ou un groupe phényle substitué par un ou plusieurs atomes d'halogène, ou par un ou plusieurs groupes alkyle en C₁-C₂ mono-, poly- ou per- fluoré.

15. Procédé selon la revendication 11, **caractérisé par le fait que** l'acide sulfonique est choisi parmi les acides halogénosulfoniques ; les acides sulfoniques aliphatiques ; les acides sulfoniques aromatiques.

16. Procédé selon **la revendication 15** , **caractérisé par le fait que** l'acide sulfonique est choisi parmi : l'acide trifluorométhanesulfonique, l'acide paratoluènesulfonique, l'acide méthanesulfonique, l'acide benzènesulfonique.

17. Procédé selon l'une des revendications 1 à 16, **caractérisé par le fait que** la quantité d'un réactif comprenant un groupe gem-dihalogéné est telle que le rapport entre le nombre de moles de réactif comprenant un groupe gemdihalogéné et le nombre de moles d'acide sulfonique est choisi entre 0,5 et 1.

18. Procédé selon l'une des revendications 1 à 17, **caractérisé par le fait que** la quantité d'un réactif comprenant deux groupes gem-dihalogénés est telle que le rapport entre le nombre de moles de réactif comprenant deux groupes gem-dihalogénés et le nombre de moles d'acide sulfonique est choisi entre 0,25 et 0,5.

19. Procédé selon l'une des revendications 1 à 18, **caractérisé par le fait que** la réaction est conduite en l'absence d'un solvant organique ou en présence d'un solvant organique inerte.

20. Procédé selon l'une des revendications 1 à 19, **caractérisé par le fait que** la réaction est menée sous pression ordinaire.

21. Procédé selon l'une des revendications 1 à 20, **caractérisé par le fait que** la réaction est conduite à une température comprise entre 100°C et 150°C.

22. Procédé selon l'une des revendications 1 à 21, **caractérisé par le fait que** l'on porte le réactif à la température réactionnelle choisie ; que l'on ajoute progressivement l'acide sulfonique puis l'on récupère l'anhydride sulfonique formé.

23. Procédé selon la revendication 22, **caractérisé par le fait que** l'on porte l'o-trichlorométhylchlorure de benzoyle mis en oeuvre en équilibre avec sa forme tautomère pseudochlorure, à la température réactionnelle choisie ; que l'on ajoute progressivement l'acide trifluorométhanesulfonique puis que l'on récupère un flux gazeux d'anhydride triflique et d'acide chlorhydrique.

24. Application du procédé décrit dans l'une des revendications 1 à 23, à la préparation de l'anhydride triflique.

## Patentansprüche

1. Verfahren zur Herstellung eines Sulfonsäureanhydrids, **dadurch gekennzeichnet, dass** es die bei einer Temperatur zwischen 80°C und 180°C durchgeführte Umsetzung einer Sulfonsäure oder eines Gemischs von zwei Sulfonsäuren mit einem Reagenz, das unter den Reaktionsbedingungen Säurepseudohalogenid-Tautomerie aufweist, wobei mindestens ein an der Tautomerie beteiligtes Kohlenstoffatom zwei Halogenatome trägt, umfasst; wobei das Reagenz die folgende Einheit im Gleichgewicht mit ihrer tautomeren Form besitzt: 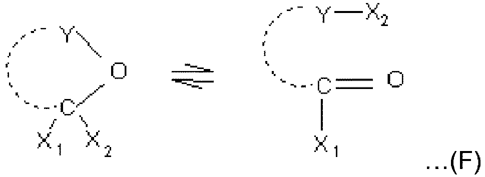 wobei in Formel (F)
- Y für eine der folgenden Gruppen steht:
. CO,
. CX₁'X₂',
- X₁ und X₂ für ein Halogenatom stehen,
- X₁' und X₂' für ein Wasserstoffatom oder ein Halogenatom stehen und höchstens eines von X₁' und X₂' für ein Wasserstoffatom steht,
- der durch die Striche symbolisierte Halbkreis für eine zweiwertige Gruppe mit 2 bis 4 Kohlenstoffatomen steht:
. bei der es sich um eine lineare Kohlenstoffkette mit einer oder zwei Doppelbindungen handelt
. oder die Teil von einem oder zwei ungesättigten oder aromatischen Ringen ist.

2. Verfahren nach Anspruch 1, **dadurch gekennzeichnet, dass** es sich bei dem Halogen um das Chloratom handelt.

3. Verfahren nach Anspruch 1, **dadurch gekennzeichnet, dass** der durch die Striche dargestellte Halbkreis in der Formel (F) für eine ethylenische Bindung oder eine 1,2-Phenylengruppe oder eine 1,2-Naphthyldiylgruppe oder eine 1,8-Naphthyldiylgruppe steht.

4. Verfahren nach Anspruch 1, **dadurch gekennzeichnet, dass** der durch die Striche dargestellte Halbkreis in der Formel (F) eine aliphatische Kohlenwasserstoffkette, in der die Halophor-Gruppe CX₁X₂ in γ-oder δ-Position zu Y steht, ist.

5. Verfahren nach einem der Ansprüche 1 bis 4, **dadurch gekennzeichnet, dass** die Reagenzien den folgenden Formeln im Gleichgewicht mit ihren tautomeren Formen entsprechen: 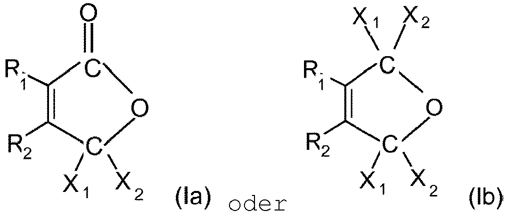 wobei in den Formeln:
- R₁ und R₂ für ein Wasserstoffatom, eine lineare oder verzweigte Alkylgruppe mit 1 bis 4 Kohlenstoffatomen oder ein Halogenatom stehen
- oder R₁ und R₂ zu einer 1,2-Phenylengruppe oder einer 1,2-Naphtyldiylguppe verknüpft sind,
- X₁ und X₂ für ein Halogenatom stehen.

6. Verfahren nach Anspruch 5, **dadurch gekennzeichnet, dass** das Reagenz der Formel (Ia) und (Ib) (und ihren tautomeren Formen) entspricht, wobei R₁ und R₂ für ein Wasserstoffatom oder eine Methylgruppe stehen oder R₁ und R₂ zu einer der folgenden Gruppen verknüpft sind:

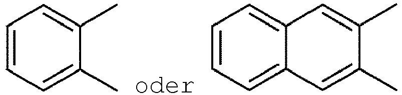

7. Verfahren nach einem der Ansprüche 5 und 6, **dadurch gekennzeichnet, dass** das Reagenz einer der folgenden Formeln entspricht:

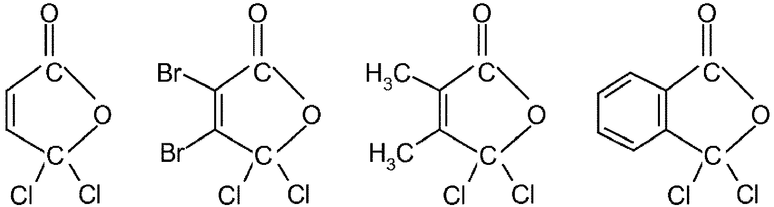

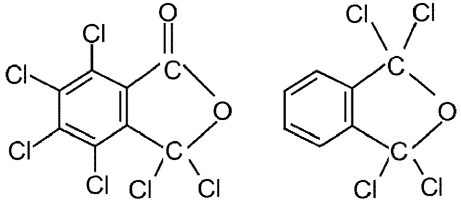

8. Verfahren nach Anspruch 1, **dadurch gekennzeichnet, dass** das Reagenz die Einheit der folgenden Formel im Gleichgewicht mit ihrer tautomeren Form umfasst: 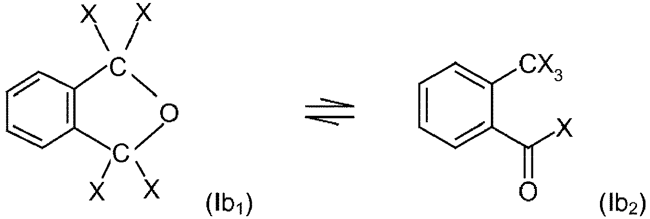 wobei in den Formeln X ein Halogenatom ist.

9. Verfahren nach Anspruch 1, **dadurch gekennzeichnet dass** das Reagenz funktionelle Gruppen Y und CX₁X₂ umfasst, die an einem Kohlenstoffatom stehen, welches zu zwei verschiedenen Benzolringen gehört, die aber ortho-kondensiert sind.

10. Verfahren nach Anspruch 9, **dadurch gekennzeichnet dass** das Reagenz der folgenden Formel entspricht:

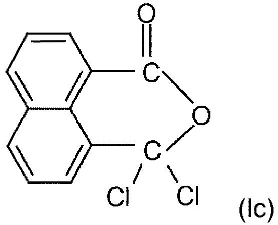

11. Verfahren nach einem der Ansprüche 1 bis 10, **dadurch gekennzeichnet, dass** die Sulfonsäure der folgenden Formel entspricht:
R_{f}-SO₃H (II)
wobei in der Formel:
- R_{f} für eine Kohlenwasserstoffgruppe mit 1 bis 20 Kohlenstoffatomen, die gegebenenfalls mindestens ein Halogenatom umfasst, steht.

12. Verfahren nach Anspruch 11, **dadurch gekennzeichnet, dass** die Kohlenwasserstoffgruppe R_{f} ausgewählt ist aus:
- einer linearen oder verzweigten Alkylgruppe mit 1 bis 12 Kohlenstoffatomen,
- einer linearen oder verzweigten Alkenyl- oder Alkinylgruppe mit 2 bis 12 Kohlenstoffatomen,
- einer Cycloalkyl- oder Cycloalkenylgruppe mit 3 bis 8 Kohlenstoffatomen,
- einer Arylgruppe mit 6 bis 20 Kohlenstoffatomen,
- eine Arylalkylgruppe mit 7 bis 20 Kohlenstoffatomen.

13. Verfahren nach Anspruch 11, **dadurch gekennzeichnet, dass** die Sulfonsäure der Formel (II) entspricht, in der R_{f} für:
- eine C₁- bis C₁₀-Alkylgruppe,
- eine mono-, poly- oder perhalogenierte C₁- bis C₁₀-Alkylgruppe mit 1 bis 23 Halogenatomen,
- eine Cycloalkylgruppe mit 3 bis 8 Kohlenstoffatomen,
- eine gegebenenfalls mono-, poly- oder perhalogenierte Cycloalkylgruppe mit 3 bis 8 Kohlenstoffatomen,
- eine Phenylgruppe,
- eine mono-, poly- oder perhalogenierte Phenylgruppe,
- eine Phenylgruppe, die durch mindestens eine gegebenenfalls mono-, poly- oder perhalogenierte C₁- bis C₁₀-Alkylgruppe oder eine Nitro- oder Nitrilgruppe substituiert ist;
- eine gegebenenfalls mono-, poly- oder perhalogenierte Arylgruppe mit 6 bis 12 Kohlenstoffatomen
steht.

14. Verfahren nach Anspruch 13, **dadurch gekennzeichnet, dass** die Sulfonsäure der Formel (II) entspricht, in der R_{f} für eine Methylgruppe, Phenylgruppe, Tolylgruppe, CF₃-Gruppe, C₄F₉-Gruppe oder eine Phenylgruppe, die durch ein oder mehrere Halogenatome oder durch eine oder mehrere mono-, poly- oder perfluorierte C₁-C₂-Alkylgruppen substituiert ist, steht.

15. Verfahren nach Anspruch 11, **dadurch gekennzeichnet, dass** die Sulfonsäure aus Halogensulfonsäuren, aliphatischen Sulfonsäuren und aromatischen Sulfonsäuren ausgewählt wird.

16. Verfahren nach Anspruch 15, **dadurch gekennzeichnet, dass** die Sulfonsäure aus Trifluormethansulfonsäure, para-Toluolsulfonsäure, Methansulfonsäure und Benzolsulfonsäure ausgewählt wird.

17. Verfahren nach einem der Ansprüche 1 bis 16, **dadurch gekennzeichnet, dass** die Menge eines Reagenzes mit einer gem-dihalogenierten Gruppe so bemessen wird, dass das Verhältnis zwischen der Zahl der Mole des Reagenzes mit einer gem-dihalogenierten Gruppe und der Zahl der Mole der Sulfonsäure zwischen 0,5 und 1 ausgewählt wird.

18. Verfahren nach einem der Ansprüche 1 bis 17, **dadurch gekennzeichnet, dass** die Menge eines Reagenzes mit zwei gem-dihalogenierten Gruppen so bemessen wird, dass das Verhältnis zwischen der Zahl der Mole des Reagenzes mit zwei gem-dihalogenierten Gruppe und der Zahl der Mole der Sulfonsäure zwischen 0,25 und 0,5 ausgewählt wird.

19. Verfahren nach einem der Ansprüche 1 bis 18, **dadurch gekennzeichnet, dass** die Umsetzung in Abwesenheit eines organischen Lösungsmittels oder in Anwesenheit eines inerten organischen Lösungsmittels durchgeführt wird.

20. Verfahren nach einem der Ansprüche 1 bis 19, **dadurch gekennzeichnet, dass** die Umsetzung unter Normaldruck geführt wird.

21. Verfahren nach einem der Ansprüche 1 bis 20, **dadurch gekennzeichnet, dass** die Umsetzung bei einer Temperatur zwischen 100°C und 150°C durchgeführt wird.

22. Verfahren nach einem der Ansprüche 1 bis 21, **dadurch gekennzeichnet, dass** man das Reagenz auf die gewählte Reaktionstemperatur bringt, die Sulfonsäure allmählich zugibt und dann das gebildete Sulfonsäureanhydrid gewinnt.

23. Verfahren nach Anspruch 22, **dadurch gekennzeichnet, dass** man im Gleichgewicht mit seiner tautomeren Pseudochlorid-Form eingesetztes oTrichlormethylbenzoylchlorid auf die gewählte Reaktionstemperatur bringt, Trifluormethansulfonsäure allmählich zugibt und dann einen Gasstrom von Trifluormethansulfonsäureanhydrid und Chlorwasserstoffsäure gewinnt.

24. Anwendung des in einem der Ansprüche 1 bis 23 beschriebenen Verfahrens zur Herstellung von Trifluormethansulfonsäureanhydrid.

## Claims

1. Process for preparing a sulfonic acid anhydride, **characterized in that** it comprises reacting, at a temperature between 80°C and 180°C, a sulfonic acid or a mixture of two sulfonic acids with a reagent exhibiting, under the reaction conditions, acid pseudohalide tautomerism, wherein at least one carbon atom engaged in the tautomerism carries two halogen atoms; said reagent having the following motif in equilibrium with its tautomeric form: 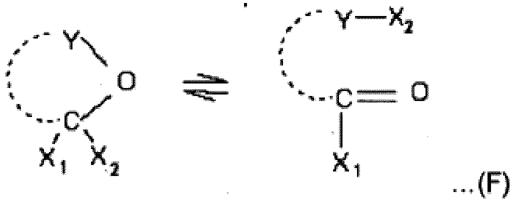 in said formula (F):
• Y represents one of the following groups:
∘ CO;
∘ CX₁' X₂' ;
• X₁, X₂ represent a halogen atom;
• X₁', X₂' represent a hydrogen atom or a halogen atom, and at most one of X₁' and X₂' represents a hydrogen atom;
• the semi-circle symbolized by the dashes represents a divalent group comprising 2 to 4 carbon atoms:
o which is a linear hydrocarbon chain comprising one or two double bonds;
∘ or which forms part of one or two unsaturated or aromatic cycles.

2. Process according to Claim 1, **characterized in that** the halogen is a chlorine atom.

3. Process according to Claim 1, **characterized in that** the semi-circle represented by the dashes in formula (F) represents an ethylene linkage or a 1,2-phenylene group or a 1,2-naphthyldiyl group or a 1,8-naphthyldiyl group.

4. Process according to Claim 1, **characterized in that** the semi-circle represented by the dashes in formula (F) is an aliphatic hydrocarbon chain in which the halophore group CX₁X₂ is in the γ or δ position with respect to Y.

5. Process according to one of Claims 1 to 4, **characterized in that** the reagents have the following formulae in equilibrium with their tautomeric forms: 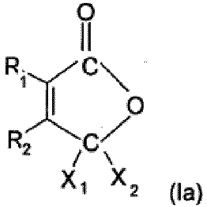 or 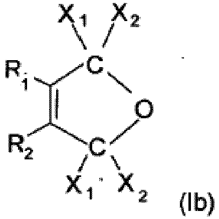 in said formulae:
• R₁, R₂ represent a hydrogen atom, a linear or branched alkyl group containing 1 to 4 carbon atoms, or a halogen atom;
• or R₁ and R₂ are linked to form a 1,2-phenylene group or a 1,2-naphthyldiyl group;
• X₁, X₂ represent a halogen atom.

6. Process according to Claim 5, **characterized in that** the reagent has formula (Ia) or (Ib) (and their tautomeric forms) in which R₁, R₂ represent a hydrogen atom or a methyl group or R₁ and R₂ are bonded to form one of the following groups: 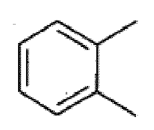 or

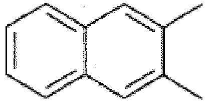

7. Process according to Claim 5 or Claim 6, **characterized in that** the reagent has one of the following formulae:

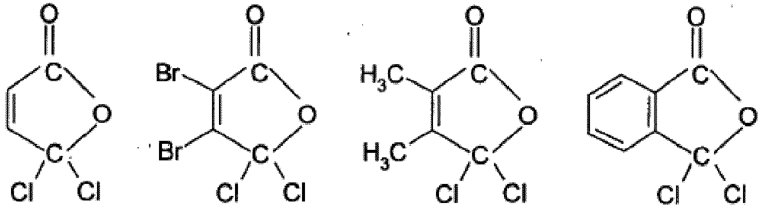

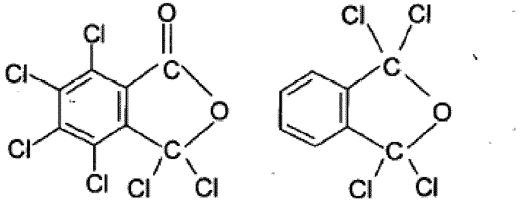

8. Process according to Claim 1, **characterized in that** the reagent comprises the motif represented by the following formula in equilibrium with its tautomeric form: 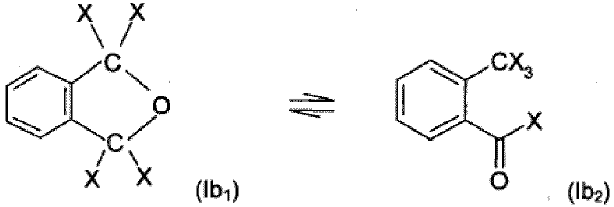 in said formulae, X is a halogen atom.

9. Process according to Claim 1, **characterized in that** the reagent comprises functional groups Y and CX₁X₂ which are carried by a carbon atom belonging to two different benzene rings but which are ortho-condensed.

10. Process according to Claim 9, **characterized in that** the reagent has the following formula:

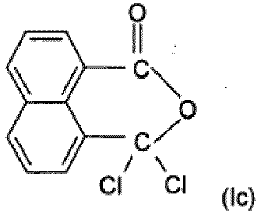

11. Process according to one of Claims 1 to 10, **characterized in that** the sulfonic acid has the following formula:
R_{f}-SO₃H (II)
in said formula:
• R_{f} represents a hydrocarbon group containing 1 to 20 carbon atoms which may or may not comprise at least one halogen atom.

12. Process according to Claim 11, **characterized in that** the hydrocarbon group R_{f} is selected from:
• a linear or branched alkyl group containing 1 to 12 carbon atoms;
• a linear or branched alkenyl or alkynyl group containing 2 to 12 carbon atoms;
• a cycloalkyl or cycloalkenyl group containing 3 to 8 carbon atoms;
• an aryl group containing 6 to 20 carbon atoms;
• an arylalkyl group containing 7 to 20 carbon atoms.

13. Process according to Claim 11, **characterized in that** the sulfonic acid has formula (II) in which R_{f} represents:
• a C₁ to C₁₀ alkyl group;
• a C₁ to C₁₀ alkyl group, mono-, poly- or per-halogenated, containing 1 to 23 halogen atoms;
• a cycloalkyl group containing 3 to 8 carbon atoms;
• a cycloalkyl group, optionally mono-, poly- or per-halogenated, containing 3 to 8 carbon atoms;
• a phenyl group;
• a mono-, poly- or per-halogenated phenyl group;
• a phenyl group substituted with at least one C₁ to C₁₀ alkyl group, optionally mono-, poly- or per-halogenated or a nitro or nitrile group;
• an aryl group, optionally mono-, poly- or per-halogenated, containing 6 to 12 carbon atoms.

14. Process according to Claim 13, **characterized in that** the sulfonic acid has formula (II) in which R_{f} represents a methyl, a phenyl, or a tolyl group, a CF₃ group, a C₄F₉ group or a phenyl group substituted with one or more halogen atoms, or with one or more mono-, poly- or per-fluorinated C₁-C₂ alkyl groups.

15. Process according to Claim 11, **characterized in that** the sulfonic acid is selected from halogenosulfonic acids; aliphatic sulfonic acids; aromatic sulfonic acids.

16. Process according to Claim 15, **characterized in that** the sulfonic acid is selected from: trifluoromethanesulfonic acid, para-toluenesulfonic acid, methanesulfonic acid and benzenesulfonic acid.

17. Process according to one of Claims 1 to 16, **characterized in that** the quantity of a reagent comprising a gem-dihalogenated group is such that the ratio between the number of moles of reagent comprising a gem-dihalogenated group and the number of moles of sulfonic acid is selected to be in the range 0.5 to 1.

18. Process according to one of Claims 1 to 17, **characterized in that** the quantity of a reagent comprising two gem-dihalogenated groups is such that the ratio between the number of moles of reagent comprising two gem-dihalogenated groups and the number of moles of sulfonic acid is selected to be in the range 0.25 to 0.5.

19. Process according to one of Claims 1 to 18, **characterized in that** the reaction is carried out in the absence of an organic solvent or in the presence of an inert organic solvent.

20. Process according to one of Claims 1 to 19, **characterized in that** the reaction is carried out at normal pressure.

21. Process according to one of Claims 1 to 20, **characterized in that** the reaction is carried out at a temperature in the range 100°C to 150°C.

22. Process according to one of Claims 1 to 21, **characterized in that** the reagent is heated to the selected reaction temperature; the sulfonic acid is added progressively, then the sulfonic anhydride formed is recovered.

23. Process according to Claim 22, **characterized in that** the benzoyl o-trichloromethyl chloride employed in equilibrium with its pseudochloride tautomer is heated to the selected reaction temperature; the trifluoromethanesulfonic acid is added progressively, then a gaseous stream of triflic anhydride and hydrochloric acid is recovered.

24. Application of the process described in one of Claims 1 to 23 to the preparation of triflic anhydride.
